# EUROPÄISCHE PATENTSCHRIFT

(11) **EP 1 166 776 B1**
(45) Veröffentlichungstag und Bekanntmachung des Hinweises auf die Patenterteilung: **02.02.2005**
(21) Anmeldenummer: 01111614.2
(22) Anmeldetag: 12.05.2001
(51) Int. Cl.: A61K 9/22

(54) **Verfahren zur Herstellung fester oraler Darreichungsformen mit retardierender Wirkstofffreisetzung**
Process of preparation of solid oral dosage forms with delayed release
Procédé de préparation de formes orales solides à libération retardée

(30) Priorität: 19.06.2000 DE 10029201
(43) Veröffentlichungstag der Anmeldung: 02.01.2002
(73) Patentinhaber: BASF AKTIENGESELLSCHAFT, 67056 Ludwigshafen (DE)
(72) Erfinder: Kolter, Karl, Dr., 67117 Limburgerhof (DE); Flick, Dieter, 67459 Böhl-Iggelheim (DE); Ascherl, Hermann, 67246 Dirmstein (DE)

(56) Entgegenhaltungen:
- EP-A- 0 868 910
- US-A- 4 801 460
- US-A- 5 585 093
- D. FLICK ET AL.: "Melt granulation with Kollidon SR for the manufacture of sustained-release tablets " CONTROLED RELEASE SOCIETY, INC. PROCEED. INTL. SYMP. CONTROL. REL. BIOACT. MATER.; 27 (2000), 7. - 13. Juli 2000, XP001119910

## Beschreibung

Die vorliegende Erfindung betrifft ein Verfahren zur Herstellung fester oraler Darreichungsformen mit retardierter Wirkstofffreisetzung, enthaltend mindestens einen Wirkstoff, eine vorformulierte Mischung aus Polyvinylacetat und Polyvinylpyrrolidon, gegebenenfalls wasserlösliche Polymere oder lipophile Zusatzstoffe sowie gegebenenfalls weitere übliche Hilfsstoffe, dadurch gekennzeichnet, daß die Granulierung dieser Mischung oder Teilen dieser Mischung durch Erwärmen auf 40°C bis 130°C erfolgt und das Granulat nach Zumischung üblicher Hilfsstoffe anschließend tablettiert wird.

Sogenannte Depot- oder Retarddarreichungsformen gewinnen besonders in der pharmazeutischen Technologie eine immer größere Bedeutung. Aufgrund der Möglichkeit, durch die Wahl geeigneter Hilfsstoffe, die Wirkstofffreisetzung zu steuern, soll die therapeutische Wirkung der Darreichungsform verbessert werden. Die Freisetzungsprinzipien reichen dabei von der verzögerten Wirkstoffauflösung, der Errichtung von Diffusionsbarrieren oder der quellungsorientierten Freigabe, bis hin zur chemisch kontrollierten Freigabe durch Bioerosion. In der Praxis findet häufig das Prinzip der Matrixtabletten Anwendung.

Die Herstellung dieser Retarddarreichungsformen, Matrixtabletten, erfolgt häufig durch Granulation und anschließende Tablettierung. Eine besondere Form der Agglomeration stellt die Schmelzgranulation dar. Im Gegensatz zur herkömmlichen Feuchtgranulation, bei der eine Durchmischung mit Lösemittel oder einer Bindemittell-Lösung befeuchtet wird, wird bei diesem Verfahren kein zusätzliches Lösungsmittel zugesetzt. Vielmehr werden bei dieser Art der Agglomeration Bindemittel eingesetzt, die bei Raumtemperatur fest und ab Temperaturen von ca. 50°C schmelzen. Der Verzicht auf zusätzliches Lösemittel ist besonders interessant, da aufgrund fehlender Trocknungsphasen die Prozesszeiten deutlich verkürzt werden und speziell bei wasserempfindlichen Wirkstoffen kein Einsatz von organischen Lösemitteln nötig ist.

Als Matrixbildner werden häufig Stoffe aus der Gruppe der Cellulosederivate eingesetzt, aber auch Stoffe aus der Gruppe der Fette und Wachse. Da diese Stoffe und auch die Wirkstoffe aufgrund ihrer physikalischen Eigenschaften jedoch häufig nur schwer oder gar nicht auf den Tablettenpressen zu verarbeiten sind, ist eine Granulierung oftmals unausweichlich.

Viele der eingesetzten Matrixbildner besitzen außerdem keine ausreichende Fähigkeit, um auch als Bindemittel zu fungieren, womit Tabletten mit einer ausreichenden mechanischen Stabilität hergestellt werden können. Dies macht deshalb häufig den Einsatz weiterer Hilfsstoffe nötig, die neben einem stabilen Granulat auch zu Tabletten mit optimalen Eigenschaften führen.

In DE 19729487 oder DE 2357503 werden Bindemittel eingesetzt, die schon geschmolzen sind oder während des Prozesses in den geschmolzenen Aggregatzustand gebracht werden (z.B. Cetylalkohol, Stearyalkohol oder Polyethylenglykol). Dies hat neben dem Nachteil der Aggregatszustandsänderung noch den Nachteil, dass auch die Tabletteneigenschaften nicht zufriedenstellend sind.

In DE 4408326 wird die Herstellung einer Retardtablette mit einem Gehalt an Diclofenac-Na beschrieben. Zur Bildung der Matrix wird die häufig eingesetzte Methylhydroxypropylcellulose verwendet, die den entscheidenden Nachteil mit sich bringt, das die Herstellung der Granulate mittels Feuchtgranulation in einer Wirbelschicht erfolgt, und somit einen Trocknungsschritt erforderlich macht. Besonders aufwendig ist hierbei die Einstellung der Wirkstofffreisetzung, da dies über eine Zweischichttablette erfolgt.

In DE 3829398 wird eine fixe Arzneimittelkombination beschrieben, bei der zwar auf die Verwendung von Füllstoffen verzichtet werden kann, aber bei der auch als Matrixbildner Stearylalkohol allein und/oder Acrylharze verwendet werden, die in einer Schmelze verarbeitet werden müssen.

EP 097 523 beschreibt die Herstellung retardierter Arzneistoffe, wobei die Wirkstoffe eine Kombination aus Salz und der freien Base darstellen. Bei diesem aufwendigen Verfahren sind mehrere Prozess-Schritte nötig, um das fertige Granulat zu erhalten. So werden die eigentlichen Granulate per herkömmlicher Feuchtgranulation hergestellt, getrocknet und erst dann mit einer geschmolzenen, hydrophoben Komponente, oder einer Mischung aus solchen Bestandteilen, in der Regel handelt es sich dabei um Fettalkohole, gecoatet.

US 5,403,593 beschreibt die Herstellung einer retardierenden Darreichungsform, bei der eine Kombination aus hydrophilen Cellulose-Polymeren und einem Granuliermedium mit einem Schmelzpunkt über 30°C eingesetzt werden. Auch hier wird deutlich, das eine Vielzahl an Hilfsstoffen nötig ist, um zum einen den gewünschten Granuliereffekt zu erreichen und zum anderen die Wirkstofffreisetzung einzustellen. Obwohl dieses Verfahren in einem Apparat durchgeführt werden kann, ist hier ein Abkühlen der Granulate auf Raumtemperatur vor der weiteren Verarbeitung nötig.

DE 4031881 beschreibt zwar das Herstellen eines Granulates, das unter anderem Polyvinylacetat als Thermoplast enthält, aber zum einen wird das Granulat aus einer Schmelze hergestellt, und zum andern dient bei diesem Verfahren der geschmolzene Wirkstoff als Lösungsmittel für den oder die Hilfsstoffe, die die Retardierung bewirken.

In US 5,169,645 wird unter anderem auch die Herstellung von Granulaten mit Wachsen dargestellt, deren Eigenschaften durch Zugabe weiterer Stoffe, wie z.B. Polyvinylacetat, beeinflusst werden können. Hier muss zum einen das Wachs aufgeschmolzen werden und zum anderen die Eigenschaften wie z.B. die der Freisetzung durch Zugabe weiterer Stoffe eingestellt werden. Ähnlich verhält es sich auch im Patent US 5,000,965, wo das Polyvinylacetat geschmolzen wird und zusätzlich mit Emulsionshilfsstoffen gemischt wird.

In DE 19729487 wird ein Verfahren zur Herstellung von Wirkstoff-Zubereitungen mit kontrollierter Freisetzung aus einer Matrix beschrieben. Dort erfolgt die Einstellung der Freisetzungscharakteristik mittels einer thermischen Nachbehandlung in der Wirbelschicht. Diese Form der Herstellung ist sehr umständlich, da ein zweiter Arbeitsgang nach dem Granulieren, das Wechseln des Arbeitsgerätes eingeschlossen, nötig ist, bei dem das Granulat nochmals solange erwärmt werden muss, bis die Schmelztemperatur des Bindemittels erreicht ist.

In EP 0204596 wird die Herstellung von Mikropartikeln durch Extrusion beschrieben. Bei diesem Verfahren ist der Zusatz von nicht hydrophilen Polymeren und einer Mischung von mindestens zwei Lipid-Bindemitteln nötig, was das gesamte Verfahren wiederum sehr aufwendig macht.

DE 3612212 beschreibt die Herstellung pharmazeutischer Formen durch Extrusion oder Spritzgießen, bei dem das schmelzbare N-Vinylpyrrolidon-Polymerisat eingesetzt wird und gegebenenfalls zusätzliche stickstoff- und/ oder sauerstoffhaltige Comonomere einpolymerisiert werden. Verfahrensbedingt kommt es hierbei allerdings zu einer kompletten Durchschmelzung der Mischung.

Die vorstehend beschriebenen Zubereitungen und Verfahren beinhalten häufig, dass sehr lipophile vollständig durchschmelzende Hilfsstoffe eingesetzt werden. Im flüssigen Aggregatzustand lösen diese sehr lipophilen Hilfsstoffe wie z.B. Wachse Wirkstoffe auf und/oder schließen sie vollständig ein. Während der Freisetzung werden daher lipophile Arzneistoffe, die eine hohe Affinität zu diesen sehr lipophilen Wirkstoffen haben, nicht mehr vollständig freigesetzt.

Generell ist immer der Nachteil vorhanden, dass sehr lipophile Bereiche existieren, die nicht durch hydrophile Polymere hydrophilisiert sind. In solche Bereiche kann daher kein Wasser eindringen.

Hinzu kommt, dass diese lipophilen Hilfsstoffe sehr schlecht pressbar sind. Es werden nur niedrige Bruchfestigkeiten erzielt, der Abrieb ist hoch und während der Herstellung tritt Kleben auf, das - wenn überhaupt - nur mit sehr grossen Mengen an Formtrennmitteln zu beheben ist.

Werden die Schmelzgranulationshilfsmittel der Pulvermischung im geschmolzenen Zustand zugesetzt, so entsteht oft das Problem der gleichmäßigen Verteilung der Schmelze im Pulver. Eine ungleichmäßige Kornstruktur, schlechte Matrizenfüllung und eine ungleichmäßige Freisetzung sind die Folge.

Obwohl schon eine Menge an Herstellungsmöglichkeiten von Retardarzneiformen bekannt sind, besteht immer noch das Bedürfnis nach einfachen, schnellen und somit kostengünstigen Verfahren, die ein unkompliziertes Arbeiten, sowohl mit wasserlöslichen als auch mit wasserunlöslichen Wirkstoffen, ermöglichen.

Aufgabe dieser Erfindung war es zum einen, wirkstoffhaltige Granulate mit guten physikalischen Eigenschaften herzustellen, die durch Tablettierung in hochdosierte pharmazeutische Darreichungsformen mit retardierter Wirkstofffreisetzung und guten mechanischen Eigenschaften gebracht werden können. Zum andern sollte das Verfahren kurze Prozess-Zeiten aufweisen, die die Herstellung der Granulate mit relativ geringem technischen Aufwand ermöglicht und sowohl für wasserempfindliche als auch für wasserunempfindliche Wirkstoffe geeignet sind, bei denen auch weitestgehend auf zusätzliche Hilfsstoffe verzichtet werden kann.

Die Aufgabe wurde erfindungsgemäß gelöst durch ein Verfahren zur Herstellung einer oralen Darreichungsform mit retardierter Wirkstofffreisetzung enthaltend
a) eine formulierte Mischung aus Polyvinylacetat und Polyvinylpyrrolidon
b) mindestens einen Wirkstoff
c) gegebenenfalls wasserlösliche Polymere oder nieder- oder hochmolekulare lipophile Zusätze
d) sowie gegebenenfalls weitere, übliche Hilfsstoffe
dadurch gekennzeichnet, daß die Granulierung der Mischung aus a) bis d) oder a) bis c) oder a) und b) und d) oder a) und b) durch Erwärmen auf 40°C bis 130°C erfolgt und das Granulat anschließend nach Zumischung üblicher Hilfsstoffe tablettiert wird.

Bei dem erfindungsgemäßen Verfahren wird das Prinzip der Schmelzgranulation angewandt und eine formulierte Mischung aus Polyvinylacetat und Polyvinylpyrrolidon dient sowohl als Bindemittel als auch als Matrixbildner, wobei sich die für die Retardierung verantwortliche Matrix erst nach dem Tablettieren ausbildet.
Die Besonderheit dieses Verfahrens liegt darin, dass bei der Granulation keine Schmelze vorliegt, sondern lediglich aufgrund der niedrigen Glasübergangstemperatur (T_{g}) des Polyvinylacetats bereits ab Temperaturen von ca. 35°C die Oberfläche des Polyvinylacetats zu kleben beginnt, und somit ein Granulationseffekt eintritt. Prinzipiell ist das Verfahren unabhängig von den physiko-chemischen Eigenschaften des Wirkstoffs. Dieser kann wasserlöslich, wasserunlöslich, sauer oder basisch oder niedrig schmelzend sein.

Gegenstand der Erfindung sind auch die nach dem erfindungsgemäßen Verfahren hergestellten oralen Darreichungsformen.

Bevorzugt werden die Darreichungsformen für pharmazeutische Wirkstoffe eingesetzt. Sie können aber auch für jeden anderen Wirkstoff, bei dem eine verzögerte Freisetzung erwünscht ist eingesetzt werden.

Der Wirkstoff oder eine Kombination aus verschiedenen Wirkstoffen wird allein oder mit wasserlöslichen oder nieder- oder hochmolekularen lipophilen Zusätzen und/oder mit üblichen Hilfsstoffen und der formulierten Mischung aus Polyvinylacetat und Polyvinylpyrrolidon vorgemischt, vorzugsweise in einem Mischer, und durch Erwärmung auf Temperaturen zwischen 40 bis 130°C, vorzugsweise in einem Bereich von 45 bis 100°C, kontinuierlich oder diskontinuierlich in dem selben Apparat granuliert. Erfindungsgemäß kann das Granulat auch mittels Extrusion oder in der Wirbelschicht hergestellt werden. Wahlweise kann das noch warme Granulat oder das schon abgekühlte Granulat über ein Zwangssieb, mit Maschenweiten zwischen 0,2 mm und 3,0 mm, gegeben werden und anschließend durch Zugabe von üblichen Tablettierhilfsstoffen, wie z.B. Füllstoffe oder Gleitmittel, zu Tabletten verpresst werden. Die Einstellung der Granulateigenschaften kann vom Fachmann unter anderem über die Parameter Temperatur und Verweilzeit vorgenommen werden. Je höher die Temperatur und die Verweilzeit, desto stärker ist in der Regel der Granulationseffekt und damit desto gröber das Korn.

Zur Erhöhung der Oberflächenfeuchte können der Darreichungsform geringe Wasser- bzw. Lösungsmittelmengen (< 5 %) zugesetzt werden.

Überraschenderweise können bei dem erfindungsgemäßen Verfahren als Mischer sowohl die vorwiegend zum Vermengen eingesetzten Doppelkonus-, Pflugschar oder V-Mischer eingesetzt werden können, als auch die üblicherweise in der pharmazeutischen Technik zur Granulation eingesetzten Sigma - Kneter, Planeten -Mischkneter, Intensivmischer oder Extruder eingesetzt werden. Die zum Anschmelzen der Oberfläche benötigte Energie kann bei den Mischern wahlweise mittels Reibungswärme oder üblicher Heizmethoden wie z.B. Mantelheizung oder Mikrowellen zugeführt werden. Als besonderer Vorteil hat sich dabei unerwarteterweise ausgezeichnet, dass wie bei bisherigen eingesetzten Verfahren eine Vorrichtung zum Kühlen nicht zwingend notwendig ist, da es sich bei diesem Verfahren um keine Schmelze im herkömmlichen Sinne handelt. Klebeeffekte und Anbackungen an Mischerwerkzeugen oder Mischerwänden treten daher nicht auf.

Durch den Zusatz an stark quellenden wasserlöslichen Polymeren oder lipophilen Zusatzstoffen kann die Freisetzung in fast beliebigen Grenzen variiert werden, bei gleichzeitig guter Fließfähigkeit der Tablettiermischung, hoher Bruchfestigkeit und niedrigem Abrieb der Tabletten. Durch den Zusatz von niedrigviskosen, nichtquellenden wasserlöslichen Polymeren, wie Polyvinylalkohole, Polyethylenglykole, Polyoxyethylen-Polyoxypropylen-Blockpolymerisate, Polyvinylpyrrolidone sowie Derivate, Vinylacetat-Vinylpyrrolidon-Copolymere, vorzugsweise Polyethylenglykolen, Polyvinylpyrrolidonen, Vinylacetat-Vinylpyrrolidon-Copolymeren oder Maltodextrinen, kann die Wirkstofffreisetzung beschleunigt werden.

Diese Zusatzstoffe werden in Konzentrationen von 1 bis 40 %, bevorzugt von 2 bis 30 % bezogen auf das Tablettengesamtgewicht eingesetzt. Dies ist erforderlich bei sehr niedrig dosierten Wirkstoffen, wo die zum Gerüstaufbau erforderliche Menge an formulierter Mischung aus Polyvinylacetat und Polyvinylpyrrolidon eine zu starke Retardierung mit sich bringt. Ferner auch bei schwerlöslichen Wirkstoffen, bei denen niedrige Mengen an Retardierungsmittel zwar zu einer verzögerten Freisetzung führen, aber der Gerüstaufbau unvollständig ist, starken Schwankungen unterliegt und die mechanische Stabilität der Tabletten unzureichend ist. Dies ist insbesondere der Fall wenn der Wirkstoff schlecht pressbar ist.

Die schlechte Fließfähigkeit des Wirkstoffes kann dann durch geringe Mengen an formulierter Mischung aus Polyvinylacetat und Polyvinylpyrrolidon nicht entscheidend verbessert werden. Die Erhöhung des Anteils an Retardierungsmittel verbessert diese Eigenschaften, führt dann aber zu einer zu stark retardierten Freisetzung. Das wasserlösliche nicht quellende Polymer beschleunigt die Freisetzung und stabilisiert diese gegen äußere Einflüsse. Auch die Reproduzierbarkeit ist sehr viel besser. Die üblichen Tablettierhilfsstoffe wie Lactose, Calciumphosphate, Sorbit, Mannit, mikrokristalline Cellulose oder Stärke sind hierzu nicht oder nicht ausreichend in der Lage. Wahrscheinlich führt eine Wechselwirkung des wasserlöslichen Polymers mit einer formulierten Mischung aus den Polymeren Polyvinylacetat und Polyvinylpyrrolidon zu der sehr stabilen, pressdruckunabhängigen und reproduzierbaren Freisetzung. Auch die Bruchfestigkeit der Tabletten und der Abrieb zeigen ausgezeichnete Werte, oft sogar höher als ohne Zumischung wasserlöslicher Polymere.

Wasserlösliche aber quellende, hochviskose Polymere führen überraschenderweise zu einer langsameren Freisetzung. Es wäre zu erwarten gewesen, daß das inerte Gerüst durch das quellende Polymer zerstört wird und der Wirkstoff schneller freigesetzt wird. Daß dies nicht eintritt liegt wahrscheinlich an der großen Elastizität der formulierten Mischung aus Polyvinylacetat und Polyvinylpyrrolidon. Die sich in den Poren des Gerüstes bildende hochviskose Lösung aus dem wasserlöslichen, quellenden Polymer blockiert diese und verlangsamt so die Diffusion des Wirkstoffes nach außen. Die Freisetzung ist häufig stärker verlangsamt als durch die beiden Komponenten alleine. Es ist eine synergistische Wirkung vorhanden. Hinzu kommt, daß auch die initiale Freisetzung durch eine Gelbildung an der Oberfläche reduziert wird und das Freisetzungsprofil dadurch "linearisiert" wird. Die mechanischen Eigenschaften der Tabletten bleiben auf einem sehr hohen Niveau.

Als wasserlösliche quellende Polymere können eingesetzt werden: Alginate, Pektine, Galactomannane, Carrageenane, Dextran, Curdlan, Pullulan Gellan, Chitin, Gelatine, Xanthane, Hemicellulosen, Cellulosederivate wie Methylcellulose, Hydroxypropylmethylcellulose, Hydroxypropylcellulose, Hydroxyethylcellulose, Carboxymethylcellulose, Stärkederivate wie Carboymethylstärke, abgebaute Stärke, Maltodextrine, Polyacrylsäure, Polymethacrylsäure, Acrylsäure-Methacrylsäure-Copolymere, Polyvinylalkohole, hochmolekulare Polyethylenglykole, Polyoxyethylen-Polyoxypropylen-Blockpolymerisate, hochmolekulare Polyvinylpyrrolidone sowie Derivate davon.

Das Verhältnis Wirkstoff zu Retardierungsmittel beträgt zwischen 5 : 95 und 85 :15.

Eine Verstärkung der Retardwirkung kann auch durch feinteilige lipophile Zusatzstoffe erfolgen. Hierbei lagern sich diese Zusatzstoffe in die Poren und Kanäle des Gerüstes aus Polyvinylacetat und Polyvinylpyrrolidon und blockieren diese. Es ist wichtig, daß diese Stoffe in kleiner Korngröße eingesetzt werden, da sie in grober Form keine bzw. nur eine geringe Wirkung entfalten. Als lipophile Zusatzstoffe können sowohl Polymere als auch niedermolekulare Verbindungen verwendet werden. Bevorzugt sind allerdings die Polymere.

Zu diesen Zusatzstoffen zählen: Cellulosederivate wie Ethylcellulose, Celluloseacetat, Celluloseacetatphthalat, Celluloseacetatsuccinat, Hydroxypropylmethylcelluloseacetatphthalat, Hydroxypropylmethylcelluloseacetatsuccinat, Acrylatester-Methacrylatester-Copolymerisate insbesondere Methylmethacrylat-Ethylacrylat-Copolymere, Ammonio-Methacrylate-Copolymer Typ A und Typ B, Methacrylsäure-Acrylsäureester-Copolymere insbesondere Methacrylsäure-Ethylacrylat-Copolymere, Fettalkohole wie Stearylalkohol, Fettsäuren wie Stearinsäure, Fettsäureester und Fettalkoholester, Glyceride, Wachse, Lecithin.

Als wasserlösliche Zusatzstoffe können die folgenden eingesetzt werden:
Polyvinylalkohole, Polyethylenglykole, Polyoxyethylen-Polyoxypropylen-Blockpolymerisate, Polyvinylpyrrolidone sowie Derivate, Vinylacetat-Vinylpyrrolidon-Copolymere, vorzugsweise Polyethylenglykolen, Polyvinylpyrrolidonen, Vinylacetat-Vinylpyrrolidon-Copolymeren oder Maltodextrinen, sowie Salze davon.

Diese Zusatzstoffe werden in Konzentrationen von 1 bis 40 %, bevorzugt von 2 bis 30 % bezogen auf das Tablettengesamtgewicht eingesetzt.

In den erfindungsgemäßen Zubereitungen liegt die formulierte Mischung aus Polyvinylacetat und Polyvinylpyrrolidon in Konzentrationen von 10 bis 80 %, vorzugsweise von 20 bis 60 % vor. Die Molekulargewichte von Polyvinylacetat und Polyvinylpyrrolidon liegen jeweils zwischen 20000 und 1000000.

Das Verhältnis von Polyvinylacetat und Polyvinylpyrrolidon in der formulierten Mischung liegt zwischen 6:4 bis 9:1, vorzugsweise 8:2. Diese Formulierung ist so ausgelegt, dass das Polyvinylpyrrolidon feinst im Polyvinylacetat verteilt ist.

Die erfindungsgemäßen Darreichungsformen umfassen orale Darreichungsformen wie Tabletten, Extrudate, Pellets oder Granulate.

Kleinere Formlinge wie beispielsweise Pellets oder Mikrotabletten können auch in Kapseln eingebracht werden.

Darreichungsformen dieser Erfindung zeichnen sich dadurch aus, dass zusätzliche Hilfsstoffe nicht zwingend notwendig sind und folglich feste Arzneiformen mit hohem Wirkstoffgehalt hergestellt werden können. Werden dennoch Hilfsstoffe verwendet, um bestimmte Eigenschaften einzustellen, so handelt es sich um Stoffe aus der Klasse der Füllstoffe wie z.B. Milchzucker, Cellulosepulver, Mannit, Calciumdiphosphat oder verschiedene Stärken, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe.

Als Schmiermittel können Stearate von Aluminium, Calcium, Magnesium und Zinn, sowie Magnesiumsilikat, Silikone und ähnliche verwendet werden.

Fließmittel können beispielsweise sein, Talk oder kolloidales Siliciumdioxid.

Bindemittel sind z.B. mikrokristalline Cellulose.

Sprengmittel können sein quervernetztes Polyvinylpyrrolidon oder quervernetzte Natriumcarboxymethylstärke. Stabilisatoren können sein Ascorbinsäure oder Tocopherol.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandiodid oder Talkum, um die Lichtdurchlässigkeit zu erhöhen und um Farbstoffe einzusparen.

Die erfindungsgemäßen Darreichungsformen können jeden Wirkstoff für den eine verzögerte Freisetzung erwünscht ist enthalten.

Bevorzugt werden als Wirkstoffe Nahrungsergänzungs- oder Zusatzstoffe, Vitamine, Mineralstoffe oder Spurenelemente, insbesondere bevorzugt aber pharmazeutische Wirkstoffe eingesetzt.

Pharmazeutische Formulierungen der oben genannten Art können durch Verarbeiten der beanspruchten Verbindungen mit pharmazeutischen Wirkstoffen nach herkömmlichen Methoden und unter Einsatz bekannter und neuer Wirkstoffe erhalten werden. Die Wirkstoffe können dabei aus jedem Indikationsgebiet kommen.

Als Beispiele seien hier die folgenden genannt:

Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytica, Betarezeptorenblocker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel.

Überraschenderweise eignet sich jeder Wirkstoff, der sich bei den vorgegebenen Temperaturen nicht zersetzt und dessen mittlere Partikelgröße in einem Bereich zwischen 20 und 700 µm liegt, vorzugsweise aber in einem Bereich zwischen 30 und 500 µm.

Die Tablettenform kann in weiten Grenzen variiert werden. So sind gewölbte, biplane, runde, kantige Tabletten herstellbar wie auch oblong- oder football-shape-Formen. Die Größe wird nach oben limitiert durch die Schluckbarkeit nach unten durch maschinenbauliche Grenzen. Übliche Tablettengrößen liegen zwischen 1 und 16 mm, vorzugsweise zwischen 2 und 13 mm Durchmesser.

Daneben lassen sich auch Zwei- oder Mehrschichttabletten herstellen, bei denen eine Schicht die gesamte Dosis an Wirkstoff enthält oder zumindest sehr wirkstoffreich ist, während die andere Schicht sehr reich ist an der Kombination Polyvinylacetat-Polyvinylpyrrolidon. Dadurch kann zusätzlich die Wirkstofffreisetzung gezielt beeinflußt werden. Es ist sogar möglich, unter Verwendung von zwei oder mehreren Wirkstoffen diese mit unterschiedlichen Geschwindigkeiten freizusetzen, indem sie völlig oder zum größten Teil getrennt in einzelne Schichten eingearbeitet werden.

Neben der formulierten Mischung aus Polyvinylacetat und Polyvinylpyrrolidon können zusätzlich retardierende Hilfsstoffe zugegeben werden. Die Zugabe kann wahlweise sowohl vor als auch nach der Granulation erfolgen.

Die Kombination der beiden Polymere Polyvinylacetat und Polyvinylpyrrolidon ermöglicht mittels des gewählten Verfahrens die Herstellung eines Granulats in einem "Eintopf-System", wobei auf den Zusatz jeglicher Lösemittel verzichtet werden kann, und weder eine zusätzliche thermische Nachbehandlung erforderlich ist, noch ein Coaten der Tabletten. Ein weiterer Vorteil liegt auch darin, dass bekanntermaßen schwer tablettierbare Wirkstoffe auf einfache Art und Weise verarbeitet werden können.

Die besonderen Vorteile der hergestellten Granulate zeigen sich direkt bei dem bekanntermaßen schwer zu verarbeitenden Paracetamol (Typ fine crystals) in den Granulateigenschaften. Aufgrund des deutlich besseren Fließverhaltens zeigt sich der erste Vorteil der aus Polyvinylacetat und Polyvinylpyrrolidon im Verhältnis 8:2 (Kollidon SR) mittels Schmelzgranulation hergestellten Granulate gegenüber den häufig verwendeten sonstigen Matrixbildnern.

**Tabelle 1**

| Fließeigenschaften von Paracetamol-Granulat | | |
|---|---|---|
| Zusammensetzung Granulat | Böschungswinkel | Auslaufzeit |
| Paracetamol/Kollidon SR (1:1) ¹ | 32,9° | 7,84s |
| Paracetamol/Methocel K 15M (1:1) ¹ | 48,2° | stockt |
| Paracetamol/Stearylalkohol (1:1) ² | 45,6° | stockt |
| Paracetamol/Kollidon SR (1:1) ³ | 34,2° | stockt |

| | | |
|---|---|---|
| ¹ Granulation im Stephan-Mischer Typ UMC5 electronic (Fa. A. Stephan u. Söhne) Parameter: 85°C (Mantelheizung), 12,5 min, 650 rpm | | |
| ² Granulation im Intensivmischer (Diosna V20) Parameter: 12,5 min | | |
| ³ Physikalische Mischung | | |

Die Bestimmung des Böschungswinkels erfolgte nach der Pfrengle-Methode gemäß DIN 53916.

Die erfindungsgemäßen Darreichungsformen weisen gute Bruchfestigkeiten und Gewichtseinheitlichkeiten der aus den Granulaten hergestellten Tabletten auf. Aus den deutlich schlechteren Granulateigenschaften der Kombination Paracetamol/Methocel K 15M resultieren die doppelt so große relative Standardabweichung der Tablettenmassen sowie die schlechten Bruchfestigkeiten. Um die Tabletteneigenschaften zu verbessern mußten daher häufig zusätzlich Bindemittel und gut pressbare Füllstoffe zugesetzt werden.

**Tabelle 2**

| Eigenschaften von Paracetamol-Tabletten | | | |
|---|---|---|---|
| Zusammensetzung Tabletten | Bruchfestigkeit | Gewicht | srel |
| Paracetamol/Kollidon SR (1:1) ¹ | 175N | 319,0 mg | 0,4 % |
| Paracetamol/Methocel K 15M (1:1) ^{1,2} | 112N | 320,5 mg | 0,8 % |
| Paracetamol/Stearylalkohol (1:1) ^{1,2} | 53N | 311,8 mg | 0,6 % |

| | | | |
|---|---|---|---|
| ¹ Tablettierung auf einer Korsch Exzenterpresse (Typ Ek0) Hilfsstoff: 0,5 % Magnesiumstearat Stempel: 10 mm, facettiert; Presskraft: 18kN | | | |
| ² zusätzliche Hilfsstoffe: 1,0 % Aerosil 200 | | | |

Die Tabletteneigenschaften (Bruchfestigkeit und Tablettengewicht) wurden mit einem Tablettenprüfautomaten der Fa. Kraemer (Typ HT-TMB) ermittelt.

Bei der Verwendung einer formulierten Mischung aus Polyvinylacetat und Polyvinylpyrrolidon werden ohne den Zusatz weiterer Füllstoffe hervorragende Tabletteneigenschaften, selbst bei schwer tablettierbaren Produkten, erzielt.

Im Gegensatz zu den herkömmlichen Methoden können mit der erfindungsgemäßen Methode durch einfaches Handling Retardtabletten hergestellt werden, die sich durch gute mechanische Eigenschaften und ein leicht einstellbares Freisetzungsverhalten auszeichnen.

### Beispiel 1

400 g einer Kollidon SR/Paracetamol Mischung, die sich aus 50 % Kollidon SR und 50 % Paracetamol zusammensetzt, wurden in einem Stephan-Mischer mit Mantelheizung vorgemischt und bei verschiedenen Temperaturen (70 bis 85°C) bei einer Drehzahl von 650 rpm verschieden lange granuliert. Anschließend wurde das noch warme Granulat über ein 1-mm-Sieb gegeben, was zu einem sehr homogenen Granulat führt. Nach dem Zumischen von 0,5 % Magnesiumstearat wurden auf einer Exzenterpresse Korsch Ek0 10-mm-Tabletten mit Facette hergestellt.

Tabelle 3 zeigt die Abhängigkeit der Granulatkorngröße von der Granuliertemperatur und der Verweilzeit, wobei erwartungsgemäß mit längerer Granulierzeit und höherer Granuliertemperatur bessere Granuliereffekte erzielt werden.

**Tabelle 3**

| Abhängigkeit der mittleren Korngröße von der Granuliertemperatur und der Granulierzeit | | | |
|---|---|---|---|
| Granulierzeit [min] | Granuliertemperatur [°C] | | |
| | 75 | 80 | 85 |
| 7,5 | 147,4 µm | 146,2 µm | 279,2 µm |
| 12,5 | 157,6 µm | 164,6 µm | 391,8 µm |
| 17,5 | 179,7 µm | 296,8 µm | 416,6 µm |

Die mittlere Korngröße wurde mittels Laserbeugung ermittelt. Angegeben ist der D[4,3]-Wert.

### Beispiel 2

400 g einer Kollidon SR/Paracetamol Mischung, die sich aus 50 % Kollidon SR und 50 % Paracetamol zusammensetzt, wurden in einem Stephan-Mischer mit Mantelheizung vorgemischt und bei ca. 80°C bei einer Drehzahl von 650 rpm 12,5 min lang granuliert. Das eingesetzte Paracetamol wurde zuvor fraktioniert, um den Einfluss der Wirkstoffkorngröße auf die Granulation zu ermitteln. Das noch warme Granulat wird über ein 1-mm-Sieb gegeben, was zu einem sehr homogenen Granulat führt. Nach dem Zumischen von 0,5 % Magnesiumstearat wurden auf einer Exzenterpresse Korsch Ek0 10-mm-Tabletten mit Facette hergestellt.

Tabelle 4 zeigt, dass selbst kleine Wirkstoffpartikel noch problemlos granuliert werden können, und das es nicht, wie eventuell vermutbar, zu einer Abpuderung der Polymerpartikel kommt und so eine Granulation verhindert wird.

**Tabelle 4**

| Abhängigkeit der mittleren Korngröße von der mittleren Korngröße des Wirkstoffs | |
|---|---|
| Mittlere Korngröße Wirkstoff [µm] | Mittlere Korngröße Granulat [µm] |
| 58,0 | 182,6 |
| 63,1 | 178,3 |
| 92,8 | 287,9 |
| 116,8 | 502,2 |
| 179,4 | 590,2 |
| 412,2 | 640,1 |
| 557,6 | 655,3 |
| 685,2 | 672,7 |
| 930,9 | 707,1 |

Die mittlere Korngröße wurde mittels Laserbeugung ermittelt. Angegeben ist der D[4,3]-Wert.

Tabelle 5 zeigt, dass trotz deutlicher Unterschiede bei der Ausgangsware die Bruchfestigkeit nur geringfügig beeinflusst wird.

**Tabelle 5**

| Abhängigkeit der Bruchfestigkeit von der mittleren Korngröße des Wirkstoffs | |
|---|---|
| Mittlere Korngröße Wirkstoff [µm] | Bruchfestigkeit [N] |
| 58,0 | 157 |
| 63,1 | 148 |
| 92,8 | 148 |
| 116,8 | 170 |
| 179,4 | 183 |
| 412,2 | 161 |
| 557,6 | 167 |
| 685,2 | 159 |
| 930,9 | 156 |

Die Bruchfestigkeit wurde mit einem Tablettenprüfautomaten der Fa. Kraemer (Typ HT-TMB) ermittelt.

Tabelle 6 zeigt die Wirkstofffreisetzung von Tabletten nach der Paddle-Methode in VE-Wasser bei 37°C über 16 h.

**Tabelle 6**

| Abhängigkeit der Wirkstoff-Freigabe von der mittleren Korngröße | | |
|---|---|---|
| Zeit [h] | Freigesetzter Wirkstoff [%] | |
| | Mittlere Korngröße Granulat = 178 µm (Wirkstoff = 63 µm) | Mittlere Korngröße Granulat = 590 µm (Wirkstoff = 179 µm) |
| 0,5 | 12,5 | 13,4 |
| 1,0 | 17,8 | 18,8 |
| 1,5 | 21,5 | 23,0 |
| 2,0 | 25,1 | 26,4 |
| 3,0 | 31,2 | 30,0 |
| 4,0 | 35,0 | 33,6 |
| 6,0 | 40,4 | 40,3 |
| 8,0 | 44,2 | 44,7 |
| 12,0 | 50,7 | 52,2 |
| 16,0 | 58,1 | 57,9 |

### Beispiel 3

400 g einer Kollidon SR/Theophyllin Mischung, die sich aus
a) 50 % Kollidon SR und 50 % Theophyllin
b) 43,75 % Kollidon SR und 56,25 % Theophyllin
c) 37,5 % Kollidon SR und 62,5 % Theophyllin
d) 25 % Kollidon SR und 75 % Theophyllin
zusammensetzt, wurden in einem Stephan-Mischer mit Mantelheizung vorgemischt und bei ca. 85°C bei einer Drehzahl von 650 rpm 12,5 min lang granuliert. Anschließend wurde das noch warme Granulat über ein 1-mm-Sieb gegeben, was jeweils zu einem homogenen Granulat führte. Nach dem Zumischen von 0,5 % Magnesiumstearat und 1 % Aerosil 200 wurden auf einer Rundlaufpresse (Korsch PH 106) 10-mm-Tabletten mit Facette hergestellt. Die Freisetzung erfolgt analog Beispiel 2.

Tabelle 7 zeigt deutlich den Einfluss der Kollidon SR-Menge sowohl auf die Wirkstofffreisetzung als auch auf die Bruchfestigkeit.

**Tabelle 7**

| Eigenschaften von Theophyllin-Tabletten | | |
|---|---|---|
| Zusammensetzung Tabletten | Bruchfestigkeit | t₅₀-Wert |
| a ¹ | 220N | >16 h |
| b ¹ | 202N | 15,2 h |
| c ¹ | 186N | 12,3 h |
| d ¹ | 153N | 11,6 h |

| | | |
|---|---|---|
| ¹ Tablettierung auf einer Korsch Rundlaufpresse (Korsch PH 106) Hilfsstoffe: 1,0 % Aerosil 200; 0,5 % Magnesiumstearat Stempel: 10 mm, facettiert; Presskraft: 18kN | | |

Die Bruchfestigkeit wurde mit einem Tablettenprüfautomaten der Fa. Kraemer (Typ HT-TMB) ermittelt. Zur Ermittlung der t₅₀-Zeit wurden die Tabletten über 16 h freigesetzt (Paddle-Methode; Prüfmedium: 0 bis 2 h: 0,1N HCl, 2 bis 16 h: Phosphatpuffer pH 6,8; Prüftemperatur: 37°C).

### Beispiel 4

400 g einer Kollidon SR/Coffein/Alginat Mischung, die sich aus 47,5 % Kollidon SR, 47,5 % Paracetamol und 5 % Alginat zusammensetzt, wurden in einem Schnellmischer mit Mantelheizung (Typ Gral Collette) vorgemischt und bei einer Temperatur von ca. 85°C bei einer granuliert. Anschließend wurde das noch warme Granulat über ein 1-mm-Sieb gegeben, was jeweils zu einem homogenen Granulat führte. Nach einer Granulierzeit von ca. 10 min wird das Granulat über ein 1-mm-Sieb gegeben und nach dem zumischen von 0,5 % Magnesiumstearat wurden auf einer Excenterpresse (Korsch Ek0) 10-mm-Tabletten mit Facette hergestellt.

Die Tabletten weisen schon bei 10kN Preßkraft eine Bruchfestigkeit von ca. 160N auf.

### Beispiel 5

Dass eine Retardwirkung erst nach dem Tablettieren erzielt wird, wurde anhand des folgenden Versuches (Kollidon SR/Paracetamol 1:1) durch die Freisetzung von
a) der physikalischen Mischung
b) den Granulaten (Stephan-Mischer: 650 rpm, 85°C, 12,5 min)
c) Tabletten hergestellt aus der physikalischen Mischung (10 mm, Facette; Presskraft: 18kN)
d) Tabletten hergestellt aus den Granulaten (10 mm, Facette; Presskraft: 18kN)
nachgewiesen.

Tabelle 8 zeigt das sowohl bei der physikalischen Mischung, als auch bei den Schmelzgranulaten keine Retardwirkung vorhanden ist. Ein Effekt ist erst nach der Tablettierung erkennbar, wobei bei der aus dem Schmelzgranulat hergestellten Tablette die Freisetzung noch stärker verzögert ist. Dieses Ergebnis zeigt, dass das erfindungsgemäße Verfahren der Schmelzgranulation mit der formulierten Mischung aus Polyvinylacetat und Polyvinylpyrrolidon, vorzugsweise im Verhältnis 8:2, den Retardierungseffekt in der Tablette deutlich verstärkt.

**Tabelle 8**

| Abhängigkeit der Wirkstoff-Freigabe von der Darreichungsform | | | | |
|---|---|---|---|---|
| Zeit [h] | Freigesetzter Wirkstoff [%] | | | |
| | Physikalische Mischung | Granulat (650 rpm, 85°C, 12,5 min) | Tablette vom Granulat¹ | Tablette von physikalischer Mischung ^{1,2} |
| 0,5 | 99,8 | 100,1 | 10,6 | 11,5 |
| 1,0 | | | 15,4 | 18,0 |
| 1,5 | | | 18,4 | 20,9 |
| 2,0 | | | 21,4 | 25,0 |
| 3,0 | | | 24,0 | 29,9 |
| 4,0 | | | 27,5 | 31,8 |
| 6,0 | | | 32,9 | 28,4 |
| 8,0 | | | 33,5 | 44,4 |
| 12,0 | | | 41,6 | 52,7 |
| 16,0 | | | 47,7 | 58,3 |

| | | | | |
|---|---|---|---|---|
| ¹ Tablettierung auf einer Korsch Exzenterpresse (Typ Ek0) Hilfsstoff: 0,5 % Magnesiumstearat Stempel: 10 mm, facettiert; Presskraft: 18kN | | | | |
| ² zusätzliche Hilfsstoffe: 1,0 % Aerosil 200 | | | | |

Die Wirkstofffreisetzung der Tabletten wurde nach der Paddle-Methode in VE-Wasser bei 37°C über 16 h durchgeführt.

### Beispiel 6

Eine Kollidon SR/Coffein Mischung, die sich aus 50 % Kollidon SR und 50 % Coffein zusammensetzt, wurde in einem Taumelmischer (Fa. Turbula, Typ T 10B) gemischt. Die Mischung wurde in einem Einschneckenextruder (Fa. Haake, Typ Rheocord 90) bei einer Temperatur von 50°C zu einer homogenen Masse geknetet. Mittels einer Schneidvorrichtung wurden die Stränge zu einem Granulat geschnitten, das nochmals über ein 1-mm-Sieb gegeben wird und nach dem zumischen von 0,5 % Magnesiumstearat auf einer Exzenterpresse (Korsch Ek0) zu 10-mm-Tabletten mit Facette verpreßt wird.

**Tabelle 9**

| Vergleich der mittleren Korngröße der physikalischen Mischung zum Granulat | |
|---|---|
| | Coffein / Kollidon SR (1:1) |
| Mittlere Korngröße physikalische Mischung | 80,23 µm |
| Mittlere Korngröße Granulat | 553,88 µm |

Die mittlere Korngröße wurde mittels Laserbeugung ermittelt. Angegeben ist der D[4,3]-Wert.

### Vergleichsbeispiel Hydroxypropylmethylcellulose

400 g einer Methocel K15M/Paracetamol Mischung, die sich aus 50 % Methocel K15M und 50 % Paracetamol zusammensetzt, wurden in einem Stephan-Mischer mit Mantelheizung vorgemischt und bei ca. 85°C bei einer Drehzahl von 650 rpm 12,5 min lang granuliert. Die noch warme Mischung wird über ein 1-mm-Sieb gegeben. Nach dem Zumischen von 0,5 % Magnesiumstearat und 1 % Aerosil 200 wurden auf einer Exzenterpresse Korsch Ek0 10-mm-Tabletten mit Facette hergestellt.

Zu dem nicht vorhandenen Granulationseffekt kommen die deutlich schlechteren Fließeigenschaften hinzu, sowie die schlechteren Tabletteneigenschaften. Durch das spröde Paracetamol kommt es anstelle einer Granulation sogar zu einer Kornverkleinerung, infolge Kristallbruches.

**Tabelle 10**

| Vergleich der Granulat- und Tabletteneigenschaften | | |
|---|---|---|
| | Paracetamol/Kollidon SR (1:1) ^{1,2} | Paracetamol/ Methocel K 15M (1:1) ^{1,2,3} |
| Korngröße D[4,3] [µm] (physik. Mischung) | 115,91 | 154,97 |
| Korngröße D[4,3] [µm] (Granulat) | 539,17 | 139,34 |
| Böschungswinkel [°] | 32,90 | 48,20 |
| Auslaufzeit [s] | 7,84 | stockt |
| Bruchfestigkeit [N] | 175,00 | 112,00 |
| Gewicht [mg] (srel [%]) | 319 (0,4) | 320,5 (0,8) |
| t₅₀-Wert [h] | >16 | 12,9 |

| | | |
|---|---|---|
| ¹ Granulation im Stephan-Mischer Typ UMC5 electronic (Fa. A. Stephan u. Söhne) Parameter: 85°C (Mantelheizung), 12,5 min, 650 rpm | | |
| ² Tablettierung auf einer Korsch Exzenterpresse (Typ Ek0) Hilfsstoff: 0,5 % Magnesiumstearat Stempel: 10 mm, facettiert; Presskraft: 18kN | | |
| ³ zusätzliche Hilfsstoffe: 1,0 % Aerosil 200 | | |

Die mittlere Korngröße wurde mittels Laserbeugung ermittelt. Angegeben ist der D[4,3]-Wert. Die Bestimmung des Böschungswinkels erfolgte nach der Pfrengle-Methode gemäß DIN 53916. Die Tabletteneigenschaften wurden mit einem Tablettenprüfautomaten der Fa. Kraemer (Typ HT-TMB) ermittelt. Zur Ermittlung der t₅₀-Zeit wurden die Tabletten über 16 h bei 37°C in VE-Wasser freigesetzt (Paddle-Methode).

### Vergleichsbeispiel Stearylalkohol

Zu dem im Intensivmischer vorgelegtem Paracetamol wird der geschmolzene Stearylalkohol zugegeben und 12,5 min granuliert. Das abgekühlte Granulat wird über ein 1-mm-Sieb gegeben. Nach dem Zumischen von 0,5 % Magnesiumstearat und 1 % Aerosil 200 wurden auf einer Exzenterpresse Korsch Ek0 10-mm-Tabletten mit Facette hergestellt.

Zu dem schlechten Fließverhalten kommen die deutlich schlechteren Tabletteneigenschaften hinzu. Das Tablettieren mit 18kN Presskraft war unter den gleichen Bedingungen wie bei dem Beispiel 1 nur bedingt möglich, da jede 2. Tablette gedeckelt aus der Matrize kommt. Die intakten Tabletten weisen eine geringe Bruchfestigkeit und eine Friabilität von 100 % auf.

**Tabelle 11**

| Vergleich der Granulat- und Tabletteneigenschaften | | |
|---|---|---|
| | Paracetamol/Kollidon SR (1:1) ^{1,3} | Paracetamol/ Stearylalkohol (1:1) ^{2,3,4} |
| Böschungswinkel [°] | 32,9 | 45,57 |
| Auslaufzeit [s] | 7,84 | stockt |
| Bruchfestigkeit [N] | 175 | 53 |
| Gewicht [mg] (srel [%]) | 319 (0,4) | 311,8 (0,6) |
| t₅₀-Wert [h] | >16 | 4,8 |

| | | |
|---|---|---|
| ¹ Granulation im Stephan-Mischer Typ UMC5 electronic (Fa. A. Stephan u. Söhne) Parameter: 85°C, 12,5 min, 650 rpm | | |
| ² Granulation im Intensivmischer (Diosna V20), 12,5 min | | |
| ³ Tablettierung auf einer Korsch Exzenterpresse (Typ Ek0) Hilfsstoff: 0,5 % Magnesiumstearat Stempel: 10 mm, facettiert; Presskraft: 18kN | | |
| ⁴ zusätzliche Hilfsstoffe: 1,0 % Aerosil 200 | | |

Die Bestimmung des Böschungswinkels erfolgte nach der Pfrengle-Methode gemäß DIN 53916. Die Tabletteneigenschaften wurden mit einem Tablettenprüfautomaten der Fa. Kraemer (Typ HT-TMB) ermittelt. Zur Ermittlung der t₅₀-Zeit wurden die Tabletten über 16 h bei 37°C in VE-Wasser freigesetzt (Paddle-Methode).

## Patentansprüche

1. Verfahren zur Herstellung einer oralen Darreichungsform mit retardierter Wirkstofffreisetzung enthaltend
a) als Retardierungsmittel eine formulierte Mischung aus Polyvinylacetat und Polyvinylpyrrolidon
b) mindestens einen Wirkstoff
c) gegebenenfalls wasserlösliche Polymere oder nieder- oder hochmolekulare lipophile Zusätze
d) sowie gegebenenfalls weitere, übliche Hilfsstoffe
**dadurch gekennzeichnet, dass** das Verhältnis Polyvinylacetat zu Polyvinylpyrrolidon 6 : 4 bis 9 : 1 beträgt, Polyvinylacetat und Polyvinylpyrrolidon jeweils ein Molekulargewicht von 20000 bis 1000000 aufweisen und die Granulierung der Mischung aus a) bis d) oder a) bis c) oder a) und b) und d) oder a) und b) ohne Zusatz jeglicher Lösungsmittel durch Erwärmen auf 40°C bis 130°C erfolgt.

2. Verfahren gemäß Anspruch 1, **dadurch gekennzeichnet, dass** die Kombination Wirkstoff :Retardierungsmittel in einem Verhältnis von 5:95 bis 85:15 eingesetzt wird.

3. Verfahren gemäß einem der Ansprüche 1 oder 2, **dadurch gekennzeichnet, dass** die Granulierung der Mischung durch Erwärmen auf 45 bis 100°C erfolgt.

4. Verfahren gemäß einem der Ansprüche 1 bis 3, **dadurch gekennzeichnet, dass** die Korngröße der eingesetzten Wirkstoffe in einem Bereich von 20 bis 700 µm liegt.

5. Verfahren gemäß einem der Ansprüche 1 bis 4, **dadurch gekennzeichnet, daß** als übliche Hilfsstoffe Füllstoffe, Spreng- und Adsorptionsmittel, Schmiermittel, Fließmittel, Farbstoffe, Stabilisatoren, Antioxidantien, Netzmittel, Konservierungsmittel, Formtrennmittel, Aromen oder Süßstoffe eingesetzt werden.

6. Verfahren gemäß einem der Ansprüche 1 bis 5, **dadurch gekennzeichnet, daß** als Hilfsstoffe Füllstoffe wie, Milchzucker, Cellulosepulver, Mannit, Calciumdiphosphat oder Stärke eingesetzt werden.

7. Verfahren gemäß einem der Ansprüche 1 bis 6, **dadurch gekennzeichnet, dass** zur Herstellung des Granulats das Verfahren der Mischergranulation, Wirbelschichtgranulation oder Extrusionsgranulation eingesetzt werden kann.

8. Verfahren gemäß einem der Ansprüche 1 bis 7, **dadurch gekennzeichnet, dass** die Herstellung sowohl kontinuierlich als auch diskontinuierlich erfolgen kann.

9. Verfahren gemäß einem der Ansprüche 1 bis 8, **dadurch gekennzeichnet, dass** die Weiterverarbeitung der Granulate, vornehmlich die Zwangssiebung sowohl im warmen Zustand, als auch im erkalteten Zustand erfolgen kann.

10. Verfahren gemäß einem der Ansprüche 1 bis 9, **dadurch gekennzeichnet, dass** neben der formulierten Mischung aus Polyvinylacetat und Polyvinylpyrrolidon weitere retardierende Hilfsstoffe vor, während oder nach der Granulation eingesetzt werden können.

11. Verfahren gemäß einem der Ansprüche 1 bis 10, **dadurch gekennzeichnet, dass** zur weiteren Modifizierung der Freisetzung wasserlösliche, wasserlösliche stark quellende oder lipophile Hilfsstoffe eingesetzt werden.

12. Verfahren gemäß einem der Ansprüche 1 bis 11, **dadurch gekennzeichnet, dass** als wasserlösliche stark quellende Stoffe Alginate, Pektine, Galactomannane, Carrageenane, Dextran, Curdlan, Pullulan Gellan, Chitin, Gelatine, Xanthane, Hemicellulosen, Cellulosederivate wie Methylcellulose, Hydroxypropylmethylcellulose, Hydroxypropylcellulose, Hydroxyethylcellulose, Carboxymethylcellulose, Stärkederivate wie Carboxymethylstärke, abgebaute Stärke, Maltodextrine, Polyacrylsäure, Polymethacrylsäure, Acrylsäure-Methacrylsäure-Copolymere, Polyvinylalkohole, hochmolekulare Polyethylenglykole, Polyoxyethylen-Polyoxypropylen-Blockpolymerisate, hochmolekulare Polyvinylpyrrolidone sowie Derivate davon eingesetzt werden.

13. Verfahren gemäß einem der Ansprüche 1 bis 12, **dadurch gekennzeichnet, dass** als lipophile Stoffe Fettalkohole wie Stearylalkohol, Fettsäuren wie Stearinsäure, Glyceride, Fettsäureester und Fettalkoholester, lipophile Polymer wie Ethylcellulose, Celluloseacetat, Acrylatester-Methacrylatester-Coploymerisate, Methacrylsäure-Acrylsäureester-Copolymere, Celluloseacetatphthalat, Celluloseacetatsuccinat, Hydroxypropylmethylcelluloeseacetatphthalat, Hydroxypropylmethylcelluloseacetatsuccinat sowie Derivate davon eingesetzt werden.

14. Verfahren gemäß einem der Ansprüche 1 bis 13, **dadurch gekennzeichnet, daß** die wasserlöslichen Polymere ausgewählt sind aus der Gruppe der: Polyvinylalkohole, Polyethylenglykole, Polyoxyethylen-Polyoxypropylen-Blockpolymerisate, Polyvinylpyrrolidone sowie Derivate, Vinylacetat-Vinylpyrrolidon-Copolymere, vorzugsweise Polyethylenglykolen, Polyvinylpyrrolidonen, Vinylacetat-Vinylpyrrolidon-Copolymeren oder Maltodextrinen, sowie Salzen davon.

15. Orale Darreichungsform enthaltend
a) als Retardierungsmittel eine formulierte Mischung aus Polyvinylacetat und Polyvinylpyrrolidon
b) mindestens einen Wirkstoff
c) gegebenenfalls wasserlösliche Polymere oder nieder- oder hochmolekulare lipophile Zusätze
d) sowie gegebenenfalls weitere, übliche Hilfsstoffe
**dadurch gekennzeichnet, dass** das Verhältnis Polyvinylacetat zu Polyvinylpyrrolidon 6 : 4 bis 9 : 1 beträgt, Polyvinylacetat und Polyvinylpyrrolidon jeweils ein Molekulargewicht von 20000 bis 1000000 aufweisen und die Granulierung der Mischung aus a) bis d) oder a) bis c) oder a) und b) und d) oder a) und b) ohne Zusatz jeglicher Lösungsmittel durch Erwärmen auf 40°C bis 130°C erfolgt.

16. Orale Darreichungsformen gemäß Anspruch 15, **dadurch gekennzeichnet, daß** sie als Wirkstoffe Nahrungsergänzungs- oder Zusatzstoffe, Vitamine, Mineralstoffe oder Spurenelemente oder pharmazeutische Wirkstoffe enthalten.

17. Orale Darreichungsformen gemäß einem der Ansprüche 15 oder 16, **dadurch gekennzeichnet, daß** sie als Wirkstoffe pharmazeutische Wirkstoffe enthalten.

18. Orale Darreichungsform gemäß einem der Ansprüche 15 bis 17, **dadurch gekennzeichnet, dass** der pharmazeutische Wirkstoff ausgewählt ist aus der Gruppe der Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytica, Betarezeptorenblocker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika,Antiemetika, Antidota, Antidiabetika, Antiarrhythmika, Antianämika, Antiallergika, Anthelmintika, Analgetika, Analeptika, Aldosteronantagonisten, Abmagerungsmittel.

19. Arzneimittel zur verzögerten Wirkstofffreisetzung, enthaltend eine orale Darreichungsform gemäß einem der Ansprüche 15 bis 18.

20. Arzneimittel zur verzögerten Wirkstofffreisetzung nach Anspruch 19, **dadurch gekennzeichnet, dass** es durch Verpressung einer oralen Darreichungsform gemäß einem der Ansprüche 15 bis 18, hergestellt wird.

21. Verwendung der oralen Darreichungsformen gemäß einem der Ansprüche 15 bis 18, zur verzögerten Wirkstofffreisetzung von Nahrungsergänzungs- oder Zusatzstoffen, Vitaminen, Mineralstoffen oder Spurenelementen.

## Claims

1. A process for producing an oral dosage form with sustained release of active ingredient, comprising
a) as release-slowing agent a formulated mixture of polyvinyl acetate and polyvinylpyrrolidone
b) at least one active ingredient
c) where appropriate water-soluble polymers or low or high molecular weight lipophilic additives
d) and, where appropriate, further, conventional excipients,
wherein the polyvinyl acetate to polyvinylpyrrolidone ratio is from 6:4 to 9:1 polyvinyl acetate and polyvinylpyrrolidone each have a molecular weight of from 20,000 to 1,000,000 and the mixture of a) to d) or a) to c) or a) and b) and d) or a) and b) is granulated without addition of any solvent by heating to from 40°C to 130°C.

2. A process as claimed in claim 1, wherein the active ingredient : release-slowing agent ratio employed in the combination is from 5:95 to 85:15.

3. A process as claimed in either of claims 1 or 4, wherein the mixture is granulated by heating to from 45 to 100°C.

4. A process as claimed in any of claims 1 to 3, wherein the particle size of the active ingredients employed is in a range from 20 to 700 µm.

5. A process as claimed in any of claims 1 to 4, wherein the conventional excipients employed are fillers, disintegrants and adsorbents, lubricants, flowability agents, dyes, stabilizers, antioxidants, wetting agents, preservatives, release agents, flavorings or sweeteners.

6. A process as claimed in any of claims 1 to 5, wherein fillers such as lactose, cellulose powder, mannitol, calcium diphosphate or starch are employed as excipients.

7. A process as claimed in any of claims 1 to 6, wherein the granules can be produced by employing the process of mixer granulation, fluidized bed granulation or extrusion granulation.

8. A process as claimed in any of claims 1 to 7, wherein production is possible both continuously and batchwise.

9. A process as claimed in any of claims 1 to 8, wherein further processing of the granules, principally the forced screening, can take place both in the hot state and in the cooled state.

10. A process as claimed in any of claims 1 to 9, wherein besides the formulated mixture of polyvinyl acetate and polyvinylpyrrolidone, it is possible to employ further release-sustaining excipients before, during or after the granulation.

11. A process as claimed in any of claims 1 to 10, wherein water-soluble, water-soluble highly swelling or lipophilic excipients are employed for further modification of release.

12. A process as claimed in any of claims 1 to 11, wherein the water-soluble highly swelling substances employed are alginates, pectins, galactomannans, carrageenans, dextran, curdlan, pullulan, gellan, chitin, gelatin, xanthans, hemicelluloses, cellulose derivatives such as methylcellulose, hydroxypropylmethylcellulose, hydroxypropylcellulose, hydroxyethylcellulose, carboxymethylcellulose, starch derivatives such as carboxymethylstarch, degraded starch, maltodextrins, polyacrylic acid, polymethacrylic acid, acrylic acid/methacrylic acid copolymers, polyvinyl alcohols, high molecular weight polyethylene glycols, polyoxyethylene/polyoxypropylene block copolymers, high molecular weight polyvinylpyrrolidones and derivatives thereof.

13. A process as claimed in any of claims 1 to 12, wherein the lipophilic substances employed are fatty alcohols such as stearyl alcohol, fatty acids such as stearic acid, glycerides, fatty acid esters and fatty alcohol esters, lipophilic polymers such as ethylcellulose, cellulose acetate, acrylate ester/methacrylate ester copolymers, methacrylic acid/acrylic ester copolymers, cellulose acetate phthalate, cellulose acetate succinate, hydroxypropylmethylcellulose acetate phthalate, hydroxypropylmethylcellulose acetate succinate and derivatives thereof.

14. A process as claimed in any of claims 1 to 13, wherein the water-soluble polymers are selected from the group of: polyvinyl alcohols, polyethylene glycols, polyoxyethylene/polyoxypropylene block copolymers, polyvinylpyrrolidones and derivatives, vinyl acetate/vinyl pyrrolidone copolymers, preferably polyethylene glycols, polyvinylpyrrolidones, vinyl acetate/vinylpyrrolidone copolymers or maltodextrins, and salts thereof.

15. An oral dosage form comprising
a) as release-slowing agent a formulated mixture of polyvinyl acetate and polyvinylpyrrolidone
b) at least one active ingredient
c) where appropriate water-soluble polymers or low or high molecular weight lipophilic additives
d) and, where appropriate, further, conventional excipients,
wherein the polyvinyl acetate to polyvinylpyrrolidone ratio is from 6:4 to 9:1 polyvinyl acetate and polyvinylpyrrolidone each have a molecular weight of from 20,000 to 1,000,000 and the mixture of a) to d) or a) to c) or a) and b) and d) or a) and b) is granulated without addition of any solvent by heating to from 40°C to 130°C.

16. An oral dosage form as claimed in claim 15, which comprises as active ingredients food supplements or additives, vitamins, minerals or trace elements or active pharmaceutical ingredients.

17. An oral dosage form as claimed in either of claim 15 or 16, which comprises active pharmaceutical ingredients as active ingredients.

18. An oral dosage form as claimed in any of claims 15 to 17, wherein the active pharmaceutical ingredient is selected from the group of benzodiazepines, antihypertensives, vitamins, cytostatics, anesthetics, neuroleptics, antidepressants, antibiotics, antimycotics, fungicides, chemotherapeutics, urologicals, platelet aggregation inhibitors, sulfonamides, spasmolytics, hormones, immunoglobulins, sera, thyroid therapeutics, psychopharmaceuticals, antiparkinson agents and other antihyperkinetics, ophthalmologicals, neuropathy products, calcium metabolism regulators, muscle relaxants, lipid-lowering agents, liver therapeutics, coronary agents, cardiac agents, immunotherapeutics, regulatory peptides and their inhibitors, hypnotics, sedatives, gynecologicals, antigout agents, fibrinolytics, enzyme products and transport proteins, enzyme inhibitors, emetics, perfusion promoters, diuretics, diagnostics, corticoids, cholinergics, biliary therapeutics, antiasthmatics, bronchospasmolytics, beta-receptor blockers, calcium channel blockers, ACE inhibitors, arteriosclerosis remedies, antiinflammatory agents, anticoagulants, antihypotensives, antihypoglycemics, antifibrinolytics, antiepileptics, antiemetics, antidotes, antidiabetics, antiarrhythmics, antianemics, antiallergics, anthelmintics, analgesics, analeptics, aldosterone antagonists, weight-reducing agents.

19. A drug product with delayed release of active ingredient, comprising an oral dosage form as claimed in any of claims 15 to 18.

20. A drug product for delayed release of active ingredient as claimed in claim 19, **characterized in that** it is produced by compression of an oral dosage form as claimed in any of claims 15 to 18.

21. The use of the oral dosage forms as claimed in any of claims 15 to 18 for the delayed release of active ingredients in the form of food supplements or additives, vitamins, minerals or trace elements.

## Revendications

1. Procédé de préparation d'une forme d'administration orale à libération retardée de la substance active, contenant
a) comme agent retard, un mélange formulé à base d'acétate de polyvinyle et de polyvinylpyrrolidone,
b) au moins une substance active,
c) éventuellement des polymères solubles dans l'eau ou des additifs lipophiles à bas ou haut poids moléculaire,
d) ainsi qu'éventuellement d'autres adjuvants courants,
**caractérisé en ce que** le rapport entre l'acétate de polyvinyle et la polyvinylpyrrolidone est de 6/4 à 9/1, **en ce que** l'acétate de polyvinyle et la polyvinylpyrrolidone présentent chacun un poids moléculaire de 20.000 à 1.000.000 et **en ce que** la granulation du mélange de a) à d) ou de a) à c) ou de a) et de b) et de d) ou de a) et de b) a lieu sans addition d'un quelconque solvant, par chauffage à 40°C jusqu'à 130°C.

2. Procédé suivant la revendication 1, **caractérisé en ce que** la combinaison substance active/agent retard est mise en oeuvre dans un rapport de 5/95 à 85/15.

3. Procédé suivant l'une des revendications 1 et 2, **caractérisé en ce que** la granulation du mélange a lieu par chauffage à 45 jusqu'à 100°C.

4. Procédé suivant l'une des revendications 1 à 3, **caractérisé en ce que** la grosseur des grains des substances actives mises en oeuvre est de l'ordre de 20 à 700 µm.

5. Procédé suivant l'une des revendications 1 à 4, **caractérisé en ce que**, comme adjuvants courants, on met en oeuvre des substances de remplissage, des agents de désintégration et d'adsorption, des lubrifiants, des agents fluidifiants, des colorants, des stabilisants, des antioxydants, des agents mouillants, des agents de conservation, des agents de démoulage, des arômes ou des édulcorants.

6. Procédé suivant l'une des revendications 1 à 5, **caractérisé en ce que**, comme adjuvants, on met en oeuvre des substances de remplissage telles que du lactose, de la poudre de cellulose, de la mannite, du diphosphate de calcium ou de l'amidon.

7. Procédé suivant l'une des revendications 1 à 6, **caractérisé en ce que**, pour la préparation du produit de granulation, on peut mettre en oeuvre le procédé de la granulation en mélangeur, de la granulation en couche turbulente ou de la granulation par extrusion.

8. Procédé suivant l'une des revendications 1 à 7, **caractérisé en ce que** la préparation peut avoir lieu aussi bien de manière continue que discontinue.

9. Procédé suivant l'une des revendications 1 à 8, **caractérisé en ce que** le traitement ultérieur des produits de granulation, surtout le tamisage forcé, peut avoir lieu non seulement à l'état chaud, mais aussi à l'état refroidi.

10. Procédé suivant l'une des revendications 1 à 9, **caractérisé en ce que**, à côté du mélange formulé d'acétate de polyvinyle et de polyvinylpyrrolidone, on peut mettre en oeuvre d'autres adjuvants retardateurs avant, pendant ou après la granulation.

11. Procédé suivant l'une des revendications 1 à 10, **caractérisé en ce que**, pour une modification supplémentaire de la libération, on met en oeuvre des adjuvants solubles dans l'eau, solubles dans l'eau et fortement gonflants ou lipophiles.

12. Procédé suivant l'une des revendications 1 à 11, **caractérisé en ce que**, comme substances solubles dans l'eau et fortement gonflantes, on met en oeuvre des alginates, des pectines, des galactomannanes, du carragheenane, du dextrane, du curdlan, du pullulane gellane, de la chitine, des gélatines, des xanthanes, des hémi-celluloses, des dérivés de cellulose, tels que de la méthylcellulose, de l'hydroxypropylméthylcellulose, de l'hydroxypropylcellulose, de l'hydroxyéthylcellulose, de la carboxyméthylcellulose, des dérivés d'amidon, tels que de l'amidon carboxyméthylique, des amidons dégradés, des maltodextrines, de l'acide polyacrylique, de l'acide polyméthacrylique, des copolymères d'acide acrylique-acide méthacrylique, des alcools polyvinyliques, des polyéthylèneglycols de haut poids moléculaire, des copolymères blocs de polyoxyéthylène-polyoxypropylène, des polyvinylpyrrolidones de haut poids moléculaire, ainsi que leurs dérivés.

13. Procédé suivant l'une des revendications 1 à 12, **caractérisé en ce que**, comme substances lipophiles, on met en oeuvre des alcools gras tels que de l'alcool stéarylique, des acides gras tels que de l'acide stéarique, des glycérides, des esters d'acides gras et des esters d'alcools gras, des polymères lipophiles tels que de l'éthylcellulose, de l'acétate de cellulose, des copolymères d'esters d'acrylate-méthacrylate, des copolymères d'esters d'acide acrylique-acide méthacrylique, de l'acétophtalate de cellulose, de l'acétosuccinate de cellulose, de l'acétophtalate d'hydroxypropylméthylcellulose, de l'acétosuccinate d'hydroxypropylméthylcellulose, ainsi que leurs dérivés.

14. Procédé suivant l'une des revendications 1 à 13, **caractérisé en ce que** les polymères solubles dans l'eau sont choisis parmi le groupe des alcools polyvinyliques, des polyéthylèneglycols, des copolymères blocs de polyoxyéthylène-polyoxypropylène, des polyvinylpyrrolidones ainsi que leurs dérivés, des copolymères d'acétate de vinyle-vinylpyrrolidone, de préférence des polyéthylèneglycols, des polyvinylpyrrolidones, des copolymères d'acétate de vinyle-vinylpyrrolidone, ou des maltodextrines, ainsi que leurs sels.

15. Forme d'administration orale contenant
a) comme agent retard, un mélange formulé à base d'acétate de polyvinyle et de polyvinylpyrrolidone,
b) au moins une substance active,
c) éventuellement des polymères solubles dans l'eau ou des additifs lipophiles à bas ou haut poids moléculaire,
d) ainsi qu'éventuellement d'autres adjuvants courants,
**caractérisée en ce que** le rapport entre l'acétate de polyvinyle et la polyvinylpyrrolidone est de 6/4 à 9/1, **en ce que** l'acétate de polyvinyle et la polyvinylpyrrolidone présentent chacun un poids moléculaire de 20.000 à 1.000.000 et **en ce que** la granulation du mélange de a) à d) ou de a) à c) ou de a) et de b) et de d) ou de a) et de b) a lieu sans addition d'un quelconque solvant, par chauffage à 40°C jusqu'à 130°C.

16. Formes d'administration orale suivant la revendication 15, **caractérisées en ce qu'**elles contiennent, comme substances actives, des compléments ou additifs nutritionnels, des vitamines, des substances minérales ou des oligoéléments ou des substances actives pharmaceutiques.

17. Formes d'administration orale suivant l'une des revendications 15 et 16, **caractérisées en ce qu'**elles contiennent, comme substances actives, des substances actives pharmaceutiques.

18. Forme d'administration orale suivant l'une des revendications 15 à 17, **caractérisée en ce que** la substance active pharmaceutique est choisie parmi le groupe des benzodiazépines, des antihypertenseurs, des vitamines, des cytostatiques, des anesthésiques, des neuroleptiques, des antidépresseurs, des antibiotiques, des antimycotiques, des fongicides, des substances chimiothérapeutiques, des produits urologiques, des inhibiteurs d'agrégation des thrombocytes, des sulfonamides, des spasmolytiques, des hormones, des immunoglobulines, des sérums, des agents thérapeutiques pour la glande thyroïde, des substances psychopharmaceutiques, des produits pour la maladie de Parkinson et d'autres antihypercinétiques, des produits ophtalmiques, des préparations de neuropathie, des régulateurs du métabolisme du calcium, des agents de relaxation des muscles, des narcotiques, des agents de diminution des lipides, des produits thérapeutiques pour le foie, des produits coronariens, des substances cardiaques, des substances immunothérapeutiques, des peptides régulateurs et de leurs substances inhibitrices, des hypnotiques, des sédatifs, des produits gynécologiques, des produits pour l'arthrite, des fibrinolytiques, des préparations enzymatiques et des protéines de transport, des inhibiteurs d'enzymes, des émétiques, des produits favorisant l'irrigation sanguine, des diurétiques, des produits de diagnostic, des corticoïdes, des substances cholinergiques, des produits thérapeutiques pour la voie biliaire, des antiasthmatiques, des broncholytiques, des bêtabloquants, des antagonistes du calcium, des inhibiteurs ACE, des produits pour l'artériosclérose, des antiphlogistiques, des anticoagulants, des antihypotoniques, des antihypoglycémiques, des antihypertoniques, des antifibrinolytiques, des antiépileptiques, des antiémétiques, des antidotes, des antidiabétiques, des antiarythmiques, des antianémiques, des anti-allergiques, des anthelminthiques, des analgésiques, des analeptiques, des antagonistes de l'aldostérone, des produits d'amaigrissement.

19. Médicament destiné à la libération retardée de substance active, contenant une forme d'administration orale suivant l'une des revendications 15 à 18.

20. Médicament destiné à la libération retardée de substance active suivant la revendication 19, **caractérisé en ce qu'**il est préparé par compression d'une forme d'administration orale suivant l'une des revendications 15 à 18.

21. Utilisation des formes d'administration orale suivant l'une des revendications 15 à 18, pour la libération retardée de compléments ou additifs nutritionnels, de vitamines, de substances minérales ou d'oligo-éléments.
